# EUROPEAN PATENT APPLICATION

(11) **EP 4 643 800 A1**
(43) Date of publication of application: **05.11.2025**
(21) Application number: 23912254.2
(22) Date of filing: 27.12.2023
(51) Int. Cl.: A61B 34/10

(54) **INFORMATION PROCESSING SYSTEM, METHOD FOR CONTROLLING INFORMATION PROCESSING SYSTEM, CONTROL PROGRAM, AND RECORDING MEDIUM**

(30) Priority: 28.12.2022 JP 2022212518
(71) Applicant: Kyocera Corporation, Kyoto-shi, Kyoto 612-8501 (JP)
(72) Inventor: WATANABE, Kenichi, Kyoto-shi, Kyoto 612-8501 (JP); KYOMOTO, Masayuki, Kyoto-shi, Kyoto 612-8501 (JP); KAMEI, Kentaro, Kyoto-shi, Kyoto 612-8501 (JP)
(74) Representative: TBK
(86) International application number: PCT/JP2023/046960
(87) International publication number: WO 2024/143476

(57) **Abstract**

To support planning of a surgical treatment suitable for an affected bone of a subject. An information processing system includes an identifier and an outputter. The identifier includes a trained first learning model. The outputter outputs at least any of instrument information, prediction information, parameter information, and implant information identified by the identifier.

## Description

### TECHNICAL FIELD

The present disclosure relates to an information processing system for supporting planning of a surgical treatment suitable for an affected bone of a subject, a method for controlling the same, and the like.

### BACKGROUND OF INVENTION

Patent Document 1 discloses a technique for proposing a surgical technique to a doctor who is going to perform a surgery on a joint part of a patient with higher accuracy than a related art.

### CITATION LIST

### PATENT LITERATURE

Patent Document 1: JP 2021-115188 A

### SUMMARY

In an aspect of the present disclosure, to resolve the above problem, an information processing system includes an acquirer, an identifier, and an outputter. The acquirer is configured to acquire input information including a medical image showing an affected bone of a subject. The identifier includes a first learning model trained using first training data. The first training data includes bone information on at least any of a bone density and a bone quality of a bone before application of a surgical treatment method including an implant embedding procedure, a shape feature, and treatment information on a surgical treatment method applied to the bone, of each of a plurality of training subjects. The outputter is configured to output at least any of instrument information, prediction information, parameter information, and implant information each identified by the identifier based on the input information. The instrument information indicates a surgical instrument to be used in a surgical treatment method suitable for the affected bone of the subject, the prediction information indicates a prediction of a state of the affected bone of the subject after application of the surgical treatment method, the parameter information indicates a parameter selectable when the surgical treatment method is applied to the affected bone of the subject, and the implant information is information on an implant embeddable in the affected bone of the subject.

In an aspect of the present disclosure, a method for controlling an information processing system includes acquiring input information including a medical image showing an affected bone of a subject, and outputting at least any of instrument information, prediction information, parameter information, and implant information each identified based on the input information and by using a first learning model trained using first training data. The instrument information indicates a surgical instrument to be used in a surgical treatment method suitable for the affected bone of the subject, the prediction information indicates a prediction of a state of the affected bone of the subject after application of the surgical treatment method, the parameter information indicates a parameter selectable when the surgical treatment method is applied to the affected bone of the subject, and the implant information is information on an implant embeddable in the affected bone of the subject. The first training data includes bone information on at least any of a bone density and a bone quality of a bone before application of a surgical treatment method including an implant embedding procedure, a shape feature, and treatment information on the surgical treatment method applied to the bone, of each of a plurality of training subjects.

The information processing system according to each aspect of the present disclosure may be implemented by a computer. In such a case, the scope of the present disclosure also includes a control program of the information processing system for causing a computer to implement the information processing system by causing the computer to operate as each component (a software element) included in the information processing system, and a computer-readable recording medium on which the control program is recorded.

### BRIEF DESCRIPTION OF THE DRAWINGS

FIG. 1 is a diagram illustrating a configuration example of an information processing system according to one aspect of the present disclosure.
FIG. 2 is a diagram illustrating a configuration example of an information processing system according to another aspect of the present disclosure.
FIG. 3 is a block diagram illustrating an example of a configuration of an information processing device according to one aspect of the present disclosure.
FIG. 4 is a diagram illustrating an example of a configuration of a first learning model executed by an identifier.
FIG. 5 is a flowchart illustrating an example of a flow of training processing by a trainer.
FIG. 6 is a flowchart illustrating an example of a flow of processing executed by the information processing device.
FIG. 7 is a block diagram illustrating an example of a configuration of an information processing device according to one aspect of the present disclosure.
FIG. 8 is a diagram illustrating a configuration example of an information processing system according to one aspect of the present disclosure.
FIG. 9 is a block diagram illustrating an example of a configuration of an information processing device according to one aspect of the present disclosure.
FIG. 10 is a diagram illustrating an example of a configuration of a learning model executed by an estimator.
FIG. 11 is a flowchart illustrating an example of a flow of training processing by a trainer.
FIG. 12 is a flowchart illustrating an example of a flow of processing executed by the information processing device.
FIG. 13 is a diagram illustrating a configuration example of an information processing system according to one aspect of the present disclosure.

### DESCRIPTION OF EMBODIMENTS

A technology for supporting planning of a surgical treatment has just begun, and thus, providing support information more suitable for a subject is one of the challenges.

One aspect of the present disclosure can support planning a surgical treatment suitable for an affected bone of a subject.

Embodiments according to the present disclosure will be described below. Hereinafter, a case in which a subject to which a surgical treatment method is to be applied is a human (that is, a "subject person") will be described in an example, but the subject is not limited to a human. That is, the "subject" according to the present disclosure may be, for example, a mammal other than a human, such as those from the equine, the feline, the canine, the bovine, or the porcine. The present disclosure also includes an embodiment in which the "subject" is reworded as "animal" when the embodiment out of the following embodiments is applicable to any of such animals.

### First Embodiment

An information processing device 1 according to one embodiment of the present disclosure is described in an example below in detail.

The information processing device 1 outputs at least any of instrument information, prediction information, parameter information, and implant information, identified by using a trained first learning model 34, based on input information including a medical image showing an affected bone of a subject. Here, the instrument information is information indicating a surgical instrument to be used in a surgical treatment method suitable for the affected bone of the subject. The prediction information is information indicating a prediction of a state of the affected bone of the subject after application of a surgical treatment method suitable for the affected bone of the subject. The parameter information is information indicating a parameter selectable when a surgical treatment method is applied to the affected bone of the subject. The implant information is information on an implant embeddable in the affected bone of the subject. The implant information may include inventory information of implants embeddable in the affected bone of the subject.

The trained first learning model 34 is trained using first training data, and the first training data includes bone information on at least any of a bone density and a bone quality of a bone before application of a surgical treatment method including an implant embedding procedure, a shape feature, and treatment information on the surgical treatment method applied to the bone, of each of a plurality of training subjects. Here, the training subjects include, for example, a patient relating to a specific disease, but may include a subject not relating to a disease, and is simply referred to as a "patient" in the following description.

The medical image may include, but not limited to, for example, at least one selected from the group consisting of an X-ray image, a computed tomography (CT) image, a magnetic resonance imaging (MRI) image, an ultrasonic image, and the like. The medical image may be, for example, an inspection apparatus image acquired from an inspection apparatus (for example, an X-ray inspection apparatus), or an image obtained by reducing noise of the inspection apparatus image. The medical image may show any of a head, a neck, a chest, a lumbar region, a hip joint, a knee joint, an ankle joint, a foot, a toe, a shoulder joint, an elbow joint, a wrist joint, a hand, and a maniphalanx. The medical image may be, for example, a dental image.

A measured value obtained by actually measuring the bone density from at least one selected from the group consisting of, for example, a hand, a lumbar spine, a proximal femur, a tibia, a heel, and an arm (for example, a radius) can be employed for the bone density of the bone. For example, a single energy X-ray absorptiometry method, a dual-energy X-ray absorptiometry (DXA) method, an ultrasound method, or a quantitative computed tomography (CT) method can be employed for the measurement of the bone density.

For example, the bone density of a patient estimated by inputting an X-ray image showing a bone of the patient to a trained estimation model machine-trained using training data including the X-ray image showing the bone and the actually measured bone density of the bone may be employed for the bone density of the bone. For example, a future bone density of the patient predicted by inputting an X-ray image showing a bone of the patient to a trained prediction model machine-trained using training data including the X-ray image showing a bone of the patient and the bone density actually measured at a time point when a predetermined period (for example, one year or three years) has elapsed since the X-ray image was captured may be employed for the bone density of the bone. The bone density of the bone may be information on the density of the bone. The bone density of the bone may be, for example, information according to definition of a brittle-bone disease guideline or may be a unique index. Information relating to the training data is employed for the bone density of the bone. For example, the bone density of the bone may be expressed by at least one selected from the group consisting of a bone mineral density per unit area (g/cm²), a bone mineral density per unit volume (g/cm³), a YAM, a T score, and a Z score. YAM is an abbreviation for "Young Adult Mean" and is sometimes called a young adult average percentage. For example, from an output layer 230, the bone density represented by the bone mineral density per unit area (g/cm²) and the bone density represented by YAM may be employed, or the bone density represented by YAM, the bone density represented by T score, and the bone density represented by Z score may be employed. The bone density of the bone may be classified (for example, a classification of SD -1.0 or greater, less than -1.0, and -2.5 or less, and YAM 80% or greater, less than 80%).

The bone quality may be based on, for example, at least one selected from the group consisting of a statistical property of the bone, a geometric property of the bone, a mechanical property of the bone, and a chemical property of the bone. The bone quality may be based on, for example, at least one selected from the group consisting of a bone metabolism marker, a gender, a race, premenopause or menopause, an age, a cortical bone state, a cancellous bone state, a trabecular bone state of a cancellous bone, disease information, bone assessment information, medication information, and presence or absence of fracture. More specifically, for example, at least one selected from the group consisting of a bone formation marker, a bone resorption marker, a bone quality marker (for example, a vitamin K value), a cortical bone thickness, a trabecular bone density, a trabecular bone direction, and a trabecular bone score of a cancellous bone can be employed for the bone quality, but the bone quality is not limited thereto. The disease information may include, for example, at least one selected from the group consisting of osteoporotics, rheumatism, osteonecrosis (such as femur head necrosis), systemic sclerosis, renal disease, osteopetrosis, and the like. The bone assessment information may include information assessed by using Fracture Risk Assessment Tool (FRAX (registered trademark)). The medication information may include, for example, at least one selected from the group consisting of a trade name, a common name, a dosage, an administration period, and an administration method (for example, oral, intravenous infusion, intramuscular infusion, or subcutaneous infusion) of a medication including at least one selected from the group consisting of a bone-resorption-suppressing medication, a bone-formation-promoting medication, and other medications (for example, a calcium preparation, a vitamin preparation, or a female hormone preparation).

The bone quality may also include, for example, a type of medullary cavity shape. For example, Dorr classification can be employed for the medullary cavity shape. The medullary cavity shape can be classified as follows using, for example, the thickness of the cortical bone and/or the shape of the medullary cavity.
- Type A: a type in which the cortical bone is thick and the medullary cavity is narrow and thin.
- Type B: a type that is intermediate between Type A and Type C and has neither a narrow nor wide medullary cavity.
- Type C: a type in which the cortical bone is thin and the medullary cavity is widened.

The shape feature includes, for example, at least a relative positional relationship, dimensions, a shape, and a general shape of a bone. In a case of an artificial hip joint, for example, the shape of the pelvis, the dimensions of the pelvis, the shape of the acetabulum of the pelvis, the dimensions of the acetabulum of the pelvis, the position of the bone head of the femur, the dimensions of the bone head of the femur, and the positional relationship between the femur and the pelvis (such as the bone head of the femur and the acetabulum of the pelvis) may be employed for the shape feature. In a case of an artificial knee joint, for example, the position of the bone head of the femur, the angle of curvature of the femur, the dimensions of the distal portion of the femur, the shape of the femur, the angle of the articular surface of the femur, the amount of the posterior offset of the femur, the dimensions of the tibia (for example, the proximal portion in contact with the tibia), the shape of the tibia (for example, the proximal portion in contact with the tibia), the dimensions of the patella, the shape of the patella, and the positional relationship between the femur and the tibia (such as the angle between the femur and the tibia) may be employed for the shape feature. In a case of a dental implant, for example, a distance between at least two of the crest of the alveolar bone, the maxillary sinus base, the nasal cavity base, the mandibular canal upper edge, and the mental foramen may be employed for the relative positional relationship between bones among the shape features.

In the present disclosure, the surgical treatment method may be at least any of an artificial joint replacement procedure or a dental implant surgery, and may be implementable by a surgical robot. In such a case, the information processing device 1 and a surgical robot control device may be communicably connected to each other, and various types of information output from the information processing device 1 may be transmitted to the surgical robot control device. In such a case, the surgical robot can be appropriately set with reference to the instrument information, the prediction information, the parameter information, and the implant information output from the information processing device 1.

More specifically, a type and/or a size of an instrument used by the surgical robot can be set as the setting of the surgical robot. Various types of parameters of an instrument used by the surgical robot during a surgery may be set. Examples of the various types of parameters include an angle at which bones (for example, a femoral neck in the case of an artificial hip joint replacement procedure and a femoral condyle, a distal femoral part, or a proximal tibial part in the case of an artificial knee joint replacement procedure) are resected, a resection amount, an insertion angle of the instrument into a human body (including, for example, a medullary cavity), an insertion depth, a rotation speed (including a torque) of the instrument, an extraction angle of the instrument, and a bone-cutting time. Such various types of parameters may be set in combination with each other, or one parameter may be set in plurality.

### Configuration of Information Processing System 100a

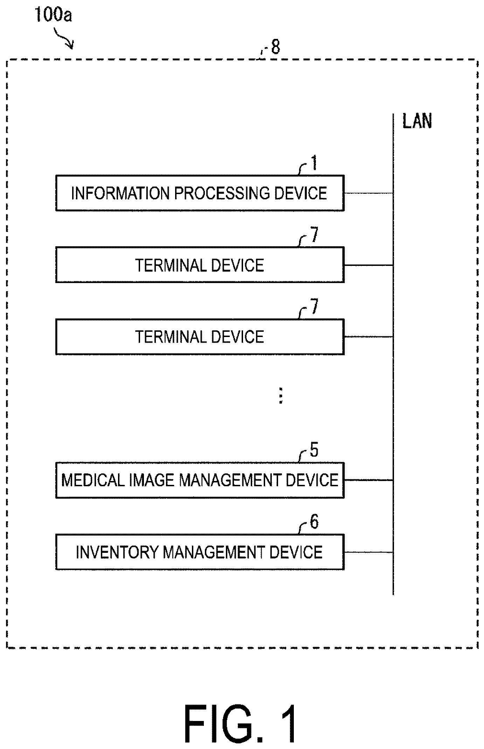

First, a configuration of an information processing system 100a according to one aspect of the present disclosure will be described with reference to FIG. 1. FIG. 1 is a diagram illustrating a configuration example of the information processing system 100a in a medical facility 8 in which the information processing device 1 is introduced.

The information processing system 100a includes the information processing device 1 and one or more terminal devices 7 communicably connected to the information processing device 1. The information processing device 1 identifies a surgical treatment method suitable for an affected bone of a subject from a medical image showing a bone of the subject, and an implant usable in the surgical treatment method. The information processing system 100a is a computer for transmitting at least any of the instrument information, the prediction information, the parameter information, and the implant information to each of the one or more terminal devices 7. The information processing device 1 according to one embodiment of the present disclosure is a device for outputting various types of information useful when a doctor or the like examines and formulates a surgical treatment method suitable for an affected bone of a subject.

The terminal device 7 functions as an outputter in the information processing system 100a, and presents information received from the information processing device 1. The terminal device 7 is a computer used by medical personnel such as a doctor and a clinical nurse (medical personnel) belonging to the medical facility 8. The terminal device 7 is, for example, a personal computer, a tablet terminal, or a smartphone. The terminal device 7 includes a communicator that transmits and receives data to and from another device, an inputter such as a keyboard and a microphone, a display capable of displaying information transmitted from the information processing device 1, an outputter such as a speaker, and the like.

An example is illustrated in which a local area network (LAN) is disposed and the information processing device 1 and the terminal devices 7 are connected to the LAN in the medical facility 8 illustrated in FIG. 1, but the present disclosure is not limited thereto. For example, the Internet, a telephone communication line network, an optical fiber communication network, a cable communication network, a satellite communication network, or the like may be applied to the network in the medical facility 8. The information processing system 100a can be adapted to a hospital information system (HIS), a radiology information system (RIS), a picture archiving and communication system (PACS) or the like in the medical setting 8. Communication in the information processing system 100a conforms to an international standard such as Digital Imaging and Communications in Medicine (DICOM).

In addition to the information processing device 1 and the terminal device 7, a medical image management device 5 and an inventory management device 6 may be communicably connected to the LAN in the medical facility 8. The medical image management device 5 is a computer functioning as a server for managing medical images taken in the medical facility 8. In such a case, the information processing device 1 may acquire a medical image showing an affected bone of the subject from the medical image management device 5. The inventory management device 6 is a computer functioning as a server for managing a holding status of implants, surgical instruments, and the like used in various types of medical procedures performed in the medical facility 8.

The information processing system 100a may include a server device (not illustrated) communicably connected to the terminal device 7, the medical image management device 5, the inventory management device 6, and the like, and the server device may have various types of functions of the information processing device 1. In such a case, the server device functions as the information processing device 1 in the information processing system 100a.

The LAN in the medical facility 8 may be communicably connected to an external communication network. In the medical facility 8, the information processing device 1 and the terminal device 7 may be directly connected to each other without going through the LAN.

### Configuration of Information Processing Device 1

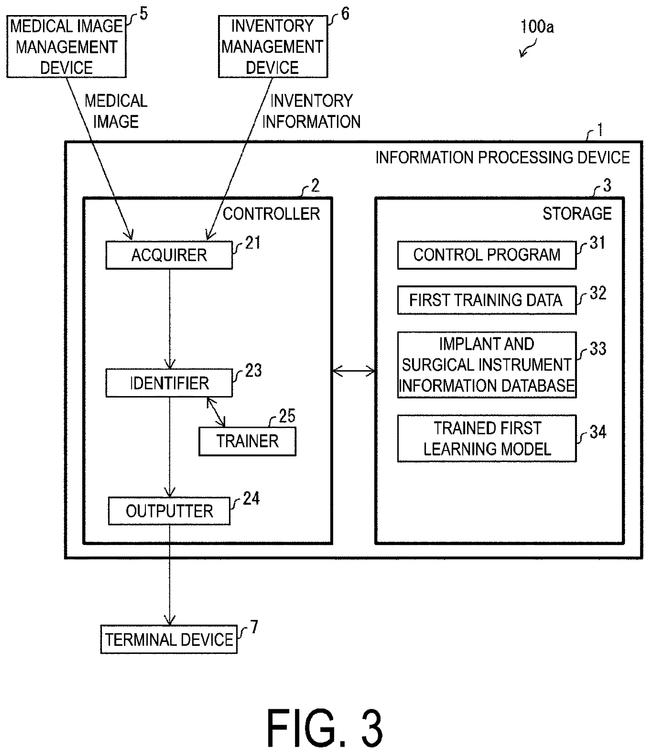

A configuration of the information processing device 1 applied to the information processing system 100a illustrated in FIG. 1 is described with reference to FIG. 3. FIG. 3 is a block diagram illustrating an example of a configuration example of the information processing device 1.

The information processing device 1 includes a controller 2 that integrally controls each component of the information processing device 1, and a storage 3 that stores various types of data to be used by the controller 2. The controller 2 includes an acquirer 21, an identifier 23, an outputter 24, and a trainer 25. The storage 3 stores a control program 31, which is a program for executing various types of controls of the information processing device 1, as well as first training data 32, an implant and surgical instrument information database 33, and the trained first learning model 34.

### Acquirer 21

The acquirer 21 acquires input information including a medical image showing an affected bone of the subject. For example, the acquirer 21 illustrated in FIG. 3 may be able to acquire, from the medical image management device 5, a medical image showing an affected bone of a subject. The input information is input data input to the identifier 23. If the surgical treatment method suitable for the affected bone of the subject includes the implant embedding procedure, the acquirer 21 may acquire the holding status of each implant and a surgical instrument to be used in the implant embedding procedure from the inventory management device 6 in the medical facility 8. The holding status is not limited to the holding status in the medical facility 8 and may be, for example, a holding status in a facility including at least one selected from the group consisting of a manufacturer, a wholesale distributor, and a distributor. The holding status may include, for example, delivery date information of delivery to the medical facility 8 from a facility including at least one selected from the group consisting of a manufacturer, a wholesale distributor, and a distributor.

### Identifier 23

The identifier 23 identifies at least any of the instrument information, the prediction information, the parameter information, and the implant information described above by inputting the input information to the trained first learning model 34. Here, the trained first learning model 34 has been subjected to learning (also referred to as training) in advance in which the first training data 32 is used. The first training data 32 is data including bone information on at least any of a bone density or a bone quality of a bone before application of a surgical treatment method including an implant embedding procedure, a shape feature, and treatment information on the surgical treatment method applied to the bone, of each of a plurality of patients.

The treatment information on the surgical treatment method as the first training data 32 is, for example, information obtained by combining at least one selected from the group consisting of the instrument information when the surgical treatment method was applied to a patient, the prediction information, the parameter information, the implant information, and therapy progress information after application of the surgical therapy. The therapy progress information includes progress information of the patient after applying the surgical treatment method. Specifically, the therapy progress information includes, for example, at least one selected from the group consisting of information on whether loosening of the implant occurred, information on whether postoperative infection of the affected area or the whole body occurred, the intensity of pain, the duration, a change in the range of motion, and information on whether re-replacement of the implant was performed, but is not limited thereto.

For example, the identifier 23 may identify a surgical treatment method suitable for the affected bone of the subject and an implant or the like embeddable in the affected bone of the subject in the surgical treatment method by inputting the input information to the trained first learning model 34. In such a case, the first training data 32 used for training the trained first learning model 34 may be data including the content and the result of the surgical treatment method applied to the affected bone of each of the plurality of patients as the treatment information. Alternatively, the first training data 32 used for training the trained first learning model 34 may be data including information indicating the surgical treatment method applied to the affected bone of each of the plurality of patients, a medical image showing the affected bone of each of the plurality of patients before application of the surgical treatment method, and information on the implant embedded in the affected bone of each of the plurality of patients.

Here, the information on the implant may be information on an artificial joint, information on a spinal implant, or information on various types of parameters of the implant determined for an embedding surgery with respect to a prosthesis or a dental implant. The artificial joint may include an artificial hip joint, an artificial knee joint, an artificial shoulder joint, an artificial elbow joint, and an artificial ankle joint. The spinal implant may include at least one selected from the group consisting of implants applied to a cervical spine, a thoracic spine, a lumbar spine, a sacral spine, and a coccygeal bone. The prosthesis may include at least one selected from the group consisting of an artificial bone (for example, in a block form or in a granular form), an artificial talus, an artificial skull, an intramedullary nail, and a bone fixation plate. The information on the implant may be, for example, at least any one selected from the group consisting of a product name of the implant, a model number thereof, a manufacturer thereof, a distributor thereof, a surface treatment thereof, and information on whether the implant is subjected to an antibacterial treatment. The information on the implant may include information on various types of parameters including at least one selected from the group consisting of a size of the implant, dimensions thereof, an angle thereof, and a material thereof.

In a case of an artificial hip joint, the information on the implant may include, for example, at least one of the following pieces of information. The information on the implant may be the following information itself, or may be at least one selected from the group consisting of a product name of the implant conforming to the following information, a model number thereof, a manufacturer thereof, a distributor thereof, and the like, but is not limited thereto.
- Information on an outer diameter of a cup.
- Information on a fixing screw of a cup.
- Information on a bone head diameter of a bone head.
- Information on a neck length of a stem.
- Information on an offset of the center of rotation of a neck from the center of rotation of a stem portion to be inserted into a medullary cavity.
- Information on a surface treatment.
- Information on a method for fixing to a bone or a biological tissue.
Here, the information on the fixing screw of the cup may be the number of fixing screws, a position of the fixing screw, a length of the fixing screw, a surface treatment of the fixing screw, a pitch of the fixing screw (for example, a pitch between the threads), a diameter of the fixing screw (the diameter of the screw portion), or the like.

In a case of an artificial knee joint, the information on the implant may include, for example, at least one of the following pieces of information. The information on the implant may be the following information itself, or may be at least one selected from the group consisting of a product name of the implant conforming to the following information, a model number thereof, a manufacturer thereof, a distributor thereof, and the like, but is not limited thereto.
- Information on the size of a femoral component
- Information on the size of a tibial component.
- Information on a tibial insert (for example, a thickness of a tibial insert).
- Information on the length of a tibial stem.
- Information on the size of a patella component.
- Information on a type of a component for each cruciate ligament treatment (for example, at least one selected from the group consisting of a posterior cruciate ligament retaining type component, a posterior cruciate ligament cutting-away type component, a posterior cruciate ligament compensating type component, and a bicruciate ligament preserving type).
- Information on implant type (for example, at least one selected from the group consisting of hinged, unilateral, mobile insert, patellofemoral joint, cemented, non-cemented, and hybrid).
- Information on a method for fixing to a bone or a biological tissue.

In a case of a spinal implant, the information on the implant may include, for example, at least one of the following pieces of information. The information on the implant may be the following information itself, or may be at least one selected from the group consisting of a product name of the implant conforming to the following information, a model number thereof, a manufacturer thereof, a distributor thereof, and the like, but is not limited thereto.
- Information on a surgical technique.
- Information on a spinal cage.
- Information on a spinal fixation plate.
- Information on a fixing screw.
- Information on a rod connecting a fixing screw.
- Information on a surface treatment.
- Information on a method for fixing to a bone or a biological tissue.

The information on the surgical technique includes, for example, information on at least one selected from the group consisting of an anterior invasive incision for making an incision on the anterior side of the body of the patient, a lateral invasive incision for making an incision on the lateral side of the body, and a posterior invasive incision for making an incision on the posterior side of the body of the patient. More specifically, the information on the surgical technique is information including, for example, an anterior lumbar interbody fusion (ALIF), a posterior lumbar interbody fusion (PLIF),
a lateral lumbar interbody fusion (LLIF), an eXtreme lumbar interbody fusion (XLIF), an oblique lumbar interbody fusion (OLIF), and a transforminal lumbar interbody fusion (TLIF).

In a case of a dental implant, the information on the implant may include, for example, at least one of the following pieces of information. The information on the implant may be the following information itself, or may be at least one selected from the group consisting of a product name of the implant conforming to the following information, a model number thereof, a manufacturer thereof, a distributor thereof, and the like, but is not limited thereto.
- Information on a shape of a tip embedded in a bone.
- Information on a surface treatment.
- Information on an intraosseous length.
- Information on a diameter.
- Information on a method for fixing to a bone or a biological tissue.

Examples of the information on a surface treatment include information on a surface treatment for on-growth of a bone on a surface, and information on a surface treatment for ingrowth of a bone in a porous body. Here, examples of the surface treatment include, but are not limited to, at least one selected from the group consisting of hydroxyapatite containing calcium hydroxide phosphate as a main component, a surface roughening treatment, Ti thermal spraying, a functional porous layer formed by three-dimensional printing, an alkali heating treatment, an anodic oxidation treatment, and an acid treatment.

The information on a method for fixing to a bone or a biological tissue may be, for example, information on a cement fixation method, a non-cement fixation method, a hybrid fixation method of the cement fixation method and the non-cement fixation method, a screw fixation method, or an overdenture mode. For example, in the case of the cement fixation method or the hybrid fixation method, the information on a method for fixing to a bone or a biological tissue may include information on an installation region of cement (for example, including at least one selected from the group consisting of an installation position, an installation range, an installation amount, and the like). In the case of a non-cement fixation method combined with reinforcement by cement, the information on a method for fixing to a bone or a biological tissue may include information on an installation region of cement.

The information on the method for fixing to the bone or the biological tissue may be, for example, information on autologous bone transplantation and/or information on artificial bone transplantation. The information on the autologous bone transplantation includes, for example, at least one selected from the group consisting of information on whether autologous bone transplantation was performed, a site where the autologous bone is obtained, an amount of the obtained autologous bone, an amount of the transplanted autologous bone, a form of the autologous bone (for example, crushed bone and/or a block), and a content of the autologous bone (for example, cancellous bone). The information on the artificial bone transplantation includes at least one selected from the group consisting of, for example, a manufacturer of the artificial bone, a material thereof, and a transplantation amount thereof. For example, in the case of an artificial hip joint, there may be a lack of bone covering the acetabulum during placement of the cup. In such a case, the information on the method for fixing to the bone or the biological tissue is information that can be a useful suggestion regarding the method for fixing to the bone or the biological tissue including at least one selected from the group consisting of the necessity, the amount, the place, and the region for transplanting the cancellous bone or the block bone.

The information on whether the implant is subjected to an antibacterial treatment includes, for example, information indicating the suitability of the implant subjected to an antibacterial treatment to a patient, information indicating the probability of occurrence of infection in the affected area, information indicating the probability of reduction of infection in the affected area, prediction of the degree of inflammation in the affected area, or information indicating the probability of occurrence of side effects when the implant subjected to an antibacterial treatment is applied. The first training data 32 may include information indicating a concentration of a blood marker relating to resistance to bacterial infection of a patient and a result of a culture test in which predetermined bacteria are cultured using blood of the patient, instead of the information on whether the implant is subjected to the antibacterial treatment.

The identifier 23 may identify the inventory information of the implant embeddable in the affected bone of the subject by referring to the holding status of the implant acquired by the acquirer 21 from the inventory management device 6. Alternatively, the identifier 23 may identify the inventory information of the surgical instrument to be used in the surgical treatment method suitable for the affected bone of the subject by referring to the holding status of the surgical instrument acquired by the acquirer 21 from the inventory management device 6.

For example, the identifier 23 may further identify the instrument information indicating the surgical instrument usable in the surgical treatment method suitable for the affected bone of the subject by inputting the input information to the trained first learning model 34. In such a case, the first training data 32 used for training the trained first learning model 34 may be data including information on the surgical instrument used in the surgical treatment method applied to the affected bone of each of the plurality of patients.

The information on the surgical instrument may be information of the surgical instrument relating to bone processing before embedding the implant. For example, when the affected area of the subject is a hip joint and the surgical treatment suitable for the affected bone of the subject includes an embedding procedure of an artificial hip joint, a reamer or a rasp, which is one of the instruments for cutting the affected bone, may be employed for a preparation for inserting a stem into the affected bone. The reamer or the rasp is an instrument for cutting the inner wall of the medullary cavity of the femur into which a stem of an artificial hip joint is inserted. For example, there may be multiple reamers or rasps with different sizes for various sizes or types of stems. That is, the information on the size (i.e., count) or the like of the stem or the rasp may be information on the surgical instrument. The information on the surgical instrument may include a size of a reamer or a rasp not corresponding to the size of the stem or the type of the stem. More specifically, the information on the surgical instrument may include an optimal size of a reamer or a rasp for the patient that is different from a size or a type of the stem.

The information on the surgical instrument may be, for example, a trial instrument used during a surgery. The trial instrument may be, for example, an instrument for checking the size of an implant or a surgical instrument or for checking the movement of an implant. In such a case, the information on the surgical instrument may be, for example, a trial instrument necessary for the surgery, or a size or a shape of the trial instrument which may fit to the patient. In an artificial hip joint replacement procedure, the trial instrument may include, for example, a neck trial, a ball trial, a broach trial, a stem trial, a plug trial, or a cup trial. In an artificial knee joint replacement procedure, the trial instrument may include, for example, a femoral trial, a plate trial, or a tibial tray trial, and a tibial stem trial.

The information on the surgical instrument may indicate, for example, that the surgical instrument suitable for the patient of the input information is not available from the same manufacturer as the manufacturer of the implant. The information on the surgical instrument may suggest a use of a custom-made surgical instrument, for example, if a surgical instrument suitable for the patient of the input information is not generally available.

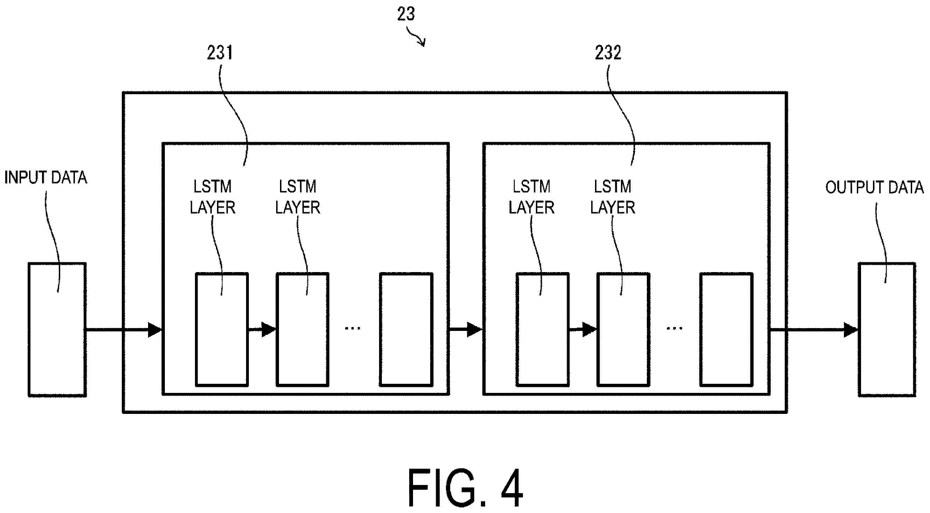

In response to the input information being input to an input layer 231 (see FIG. 4), the identifier 23 performs an operation, based on the trained first learning model 34, and outputs a surgical treatment method suitable for the affected bone of the subject and an implant usable in the surgical treatment method from an output layer 232 (see FIG. 4). As an example, the identifier 23 may be configured to extract a feature amount from the input information and use the feature amount as input data. In extracting the feature amount, known algorithms as listed below may be applied.
- Convolutional neural network (CNN).
- Auto encoder.
- Recurrent neural network (RNN).
- Long short-term memory (LSTM).
- Convolutional long short-term memory (ConvLSTM).

The trained first learning model 34 is an operation model used by the identifier 23 in performing an operation, based on the input data. The trained first learning model 34 is generated by the trainer 25 executing machine learning using the first training data 32, which will be described below, on an untrained neural network. Here, the trained first learning model 34 may also be applied to an animal other than a human. In such a case, the "patient" in the first training data 32 may be a biological species of the same type as the "subject". That is, the information processing device 1 according to the present disclosure can also identify a surgical treatment method suitable for an affected bone of an animal other than a human. A specific example of the first training data 32, a configuration of the neural network, and the training processing will be described below.

### Outputter 24

The outputter 24 transmits each piece of information identified by the identifier 23 to the terminal device 7. The outputter 24 may extract information on the implant identified by the identifier 23 from the implant and surgical instrument information database 33. The information processing device 1 may include a display (not illustrated). In such a case, the outputter 24 displays each piece of information described above on the display.

### Trainer 25

The trainer 25 controls training processing for the untrained neural network. The trainer 25 creates a trained neural network functioning as the identifier 23 by executing training processing on an untrained neural network. The first training data 32 (described below) is used for such a training. A specific example of training executed by the trainer 25 will be described below.

### Configuration of Identifier 23

A configuration of the identifier 23 will be described below with reference to FIG. 4. The configuration illustrated in FIG. 4 is merely an example and the configuration of the identifier 23 is not limited thereto.

As illustrated in FIG. 4, the identifier 23 performs operations based on the trained first learning model 34 on the input data input to the input layer 231 to output output data from the output layer 232. The output data is at least any of instrument information, prediction information, parameter information, and implant information.

The identifier 23 in FIG. 4 includes a neural network including the input layer 231 and the output layer 232. FIG. 4 illustrates a case where the neural network is an LSTM; however, the neural network is not limited thereto. For example, the neural network may be a ConvLSTM network in which CNN and LSTM are combined. The input layer 231 can extract a feature amount for a change in the input data. The output layer 232 can calculate a new feature amount, based on the feature amount extracted in the input layer 231 and the temporal change of and the initial value of the input data. Each of the input layer 231 and the output layer 232 includes a plurality of LSTM layers. Each of the input layer 231 and the output layer 232 may include three or more LSTM layers.

### Training Processing by Trainer 25

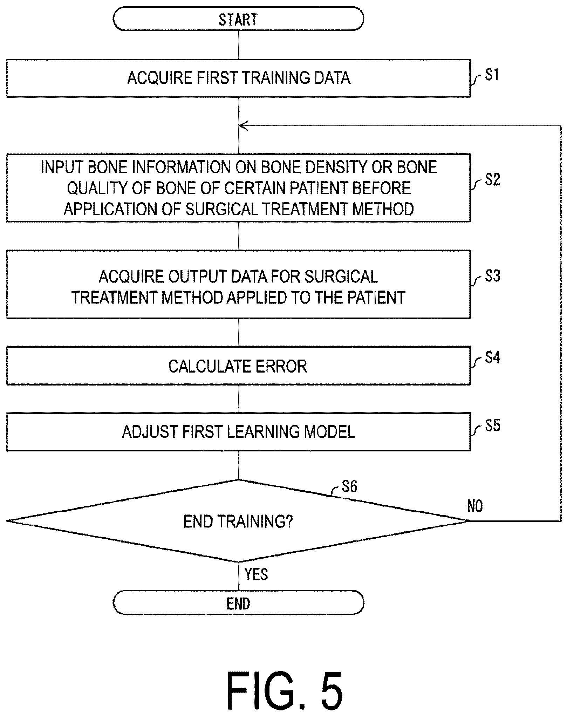

Training processing for generating the trained first learning model 34 will be described below with reference to FIG. 5. FIG. 5 is a flowchart illustrating an example of a flow of training processing by the trainer 25.

The trainer 25 acquires the first training data 32 from the storage 3 (step S1). The first training data 32 includes an explanatory variable and an objective variable to be input to the input layer 231. Here, the explanatory variable is the bone information relating to at least any of the bone density and the bone quality of the bone before application of the surgical treatment method including the implant embedding procedure, and the shape feature, of each of the plurality of patients, and the objective variable is the treatment information on the surgical treatment method applied to the bone. The bone information on at least any of the bone density and the bone quality of the bone before application of the surgical treatment method including the implant embedding procedure may be information indicating a measurement result of a bone metabolism marker or may be information indicating the bone density estimated based on an X-ray image. The bone information may include, for example, at least one selected from the group consisting of measurement results of a bone mass, a bone density, a trabecular number, a trabecular gap, a connectivity density of trabeculae, a trabecular bone score of a cancellous bone, and a bone metabolism marker.

The trainer 25 inputs the bone information of a certain patient (referred to as a patient A) to which a surgical treatment method was applied, to the input layer 231 (step S2).

The trainer 25 acquires output data regarding the surgical treatment method applied to the bone of the patient A, from the output layer 232 (step S3). The output data contains the same contents as the objective variable of the first training data 32. In FIG. 5, the order of step S2 and step S3 may be reversed. Alternatively, in FIG. 5, step S2 and step S3 may be executed simultaneously.

The trainer 25 acquires an objective variable for the patient A included in the first training data 32. The trainer 25 compares the output data acquired in step S3 with the objective variable for the patient A, calculates an error (step S4), and adjusts the first learning model being trained so as to reduce the error (step S5).

Any known method is applicable to the adjustment of the first learning model being trained. For example, an error backpropagation method may be employed as a method for adjusting the first learning model. The adjusted first learning model becomes a new first learning model, and in subsequent operations, the identifier 23 uses the new first learning model. In an adjustment stage of the first learning model, the parameters (for example, a filter coefficient and a weighting factor) used in the identifier 23 may be adjusted.

If the error is not within a predetermined range, and if explanatory variables for all the patients contained in the first training data 32 are not input (NO in step S6), the trainer 25 returns to step S2 and repeats the training processing. If the error is within the predetermined range, and if the explanatory variables for all the patients contained in the first training data 32 are input (YES in step S6), the trainer 25 ends the training processing.

When the training processing as described above is adopted, the identifier 23 can identify at least any of the instrument information, the prediction information, the parameter information, and the implant information, which are the information on the surgical treatment method suitable for the affected bone of the subject, from the input information including the medical image showing the affected bone of the subject.

### First Variation

The first training data 32 may further include at least any of event information on an event that occurred during an implant embedding procedure for each bone of a plurality of patients and procedure information on a procedure performed in the implant embedding procedure. By using such first training data 32, the trained first learning model 34 can output an event that may occur when an implant embedding procedure is performed on a subject, a procedure to be performed, and the like, from a medical image showing a bone of the subject.

Here, the event information may be information on any one or more of bone fracture, loosening, implant breakage, a range of motion, and an infection during an implant embedding procedure. The information on the bone fracture may be, for example, information on whether the affected bone was cracked due to insertion of a stem into the medullary cavity of the bone cut by a reamer or a rasp. Alternatively, the information on the bone fracture may be, for example, information on whether the bone was broken due to insertion of a rasp with a certain count into the medullary cavity of the bone of the subject. The event information may include information on the size of the reamer or the rasp used in the cutting processing when the bone was cracked.

The procedure information may be information on an impact in embedding an implant, a time required for a surgery, an amount of bleeding, a blood transfusion volume, use of bone cement, a type of bone cement, an application position of bone cement, use of antibiotics, and a treatment such as measures against infectious diseases and hemostasis. The information on impact may include, for example, the number of impacts, an instantaneous maximum load, an impact sound, a type of impactor, and a type of hammer. For a ceramic component, it may be output that a resin hammer is used.

According to such a configuration, the information processing device 1 can provide, to a doctor or the like, in advance, matters to be noted in formulating a surgical treatment method to be applied to a subject. As a result, the information processing device 1 of the subject can support the planning of the surgical treatment suitable for the affected bone of the subject to a higher degree.

### Second Variation

The adjustment of the trained first learning model 34 may be executed in another computer different from the information processing device 1. In such a case, the trained first learning model 34 may be installed in and then used by the information processing device 1. That is, in the information processing device 1, the trainer 25 is not an essential component.

### Processing Executed by Information Processing Device 1

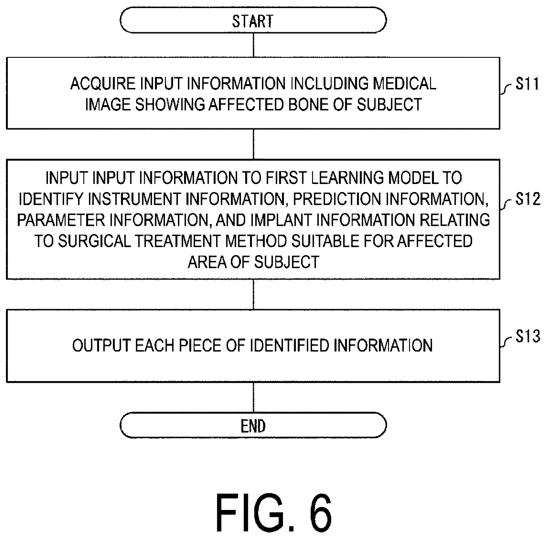

A flow of processing executed by the information processing device 1 will be described below with reference to FIG. 6. FIG. 6 is a flowchart illustrating an example of a flow of processing executed by the information processing device 1. FIG. 6 illustrates an example of processing performed when the information processing device 1 outputs the following pieces of information from the input information, but the information processing device 1 may be configured to output at least one of such pieces of information.
- Instrument information indicating a surgical instrument to be used in a surgical treatment method suitable for an affected bone of a subject.
- Prediction information indicating a prediction of a state of an affected bone of a subject after applying a surgical treatment method.
- Parameter information indicating a parameter selectable in applying a surgical treatment method to an affected bone of a subject.
- Implant information on an implant embeddable in an affected bone of a subject.

First, the acquirer 21 acquires input information including a medical image showing an affected bone of a subject (step S11: acquiring step).

The identifier 23 inputs the input information to the trained first learning model 34 to identify the instrument information, the prediction information, the parameter information, and the implant information which relate to the surgical treatment method suitable for the affected bone of the subject (step S12: identifying step).

The outputter 24 outputs each piece of information identified in step S12 (step S13: outputting step).

According to such a configuration, the information processing device 1 and the information processing system 100a can output useful information for examining and determining a surgical treatment method suitable for affected bone of a subject. All of the instrument information, the prediction information, the parameter information, and the implant information, output from the information processing device 1 are important information for a doctor or the like to examine a surgical treatment method suitable for the affected bone of the subject. For example, depending on the holding status of the surgical instrument and the implant, there is a possibility that application of the surgical treatment method to the subject needs to be postponed until delivery of the implant to be used, a possibility that the schedule for applying the surgical treatment method to a person different from the subject needs to be adjusted, or the like. The information processing device 1 and the information processing system 100a output the information as described above to support the planning of the surgical treatment suitable for the affected bones of the subject. Depending on the bone quality, the osteotomy may be difficult (for example, osteopetrosis). Therefore, for example, an expected osteotomy time or the like may be output.

### Second Embodiment

Another embodiment of the present disclosure will be described below. For convenience of description, members having the same functions as those of the members described in the above-described embodiment are denoted by the same reference signs, and description thereof is not repeated.

The information processing system 100a may be configured to generate, from the medical image included in the input information, the first predicted image that is generated from the medical image included in the input information and that shows an expected state of the affected bone of the subject after application of the surgical treatment method to the affected bone of the subject. The information processing system 100a may be configured to output parameter information including a surgical parameter being estimated from a result of image analysis of the first predicted image and being selectable by a medical personnel applying, to the subject, a surgical treatment method suitable for the affected bone of the subject.

Examples of the parameter information may include an angle at which bones (for example, a femoral neck in a case of an artificial hip joint replacement procedure, and a femoral condyle, a distal femoral part, or a proximal tibial part in a case of an artificial knee joint replacement procedure) are resected, a resection amount, an insertion angle of the instrument into a human body (including, for example, a medullary cavity), an insertion depth, a rotation speed (including a torque) of the instrument, an extraction angle of the instrument, a proposal for removing a bone spicule, a method for removing a bone spicule (for example, a resection amount and a resection range), and a cutting time of a bone. Such parameter information may be set in combination with each other, or one parameter may be set in plurality.

### Configuration of Information Processing Device 1a

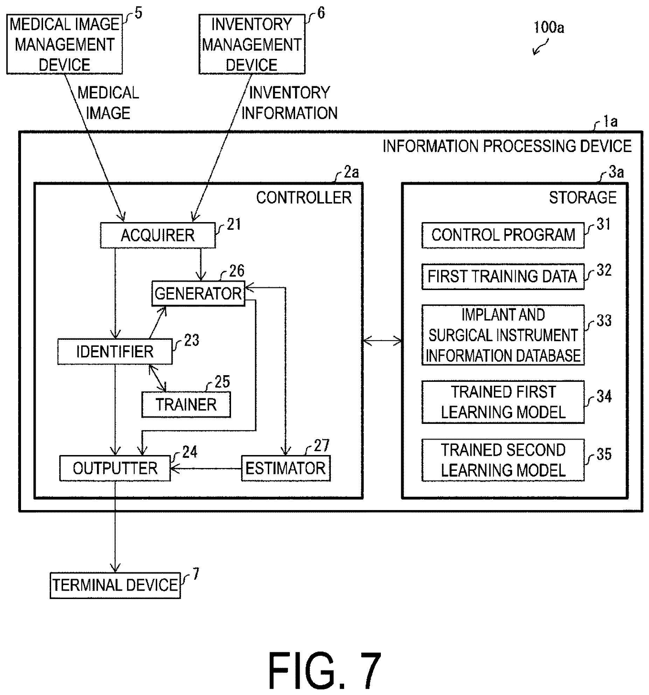

A configuration of an information processing device 1a having such a configuration will be described with reference to FIG. 7. FIG. 7 is a block diagram illustrating an example of a configuration example of the information processing device 1a. The information processing device 1a can be applied to the information processing system 100a, and information processing systems 100b, 100c, and 100d.

The information processing device 1a includes a controller 2a that integrally controls each component of the information processing device 1a, and a storage 3a that stores various types of data to be used by the controller 2a. The controller 2a includes a generator 26 and an estimator 27 in addition to the acquirer 21, the identifier 23, the outputter 24, and the trainer 25. The storage 3a stores the control program 31, which is a program for executing various types of controls of the information processing device 1a, the first training data 32, the implant and surgical instrument information database 33, and the trained first learning model 34, and further stores a trained second learning model 35.

The generator 26 generates a first predicted image from the medical image included in the input information using the second learning model 35 trained using second training data. Here, the second training data is data including a medical image obtained by imaging an affected bone of a patient to which a surgical treatment method suitable for the affected bone of the subject is applied, before and after application of the surgical treatment method. The outputter 24 transmits, to the terminal device 7, the generated first predicted image. Here, the first predicted image may be an image obtained by converting a medical image (two-dimensional image) included in the input information into a three-dimensional image using a known image conversion method.

By checking the first predicted image before actually applying the surgical treatment method to the subject, the doctor or the like can determine whether the surgical treatment method suitable for the affected bone of the subject is actually suitable for the subject. As a result, the information processing device 1a can support the application of the surgical treatment method more suitable for the affected bones of the subject.

The estimator 27 may estimate a surgical parameter selectable by a doctor or the like applying the surgical treatment method to the subject from a result of image analysis of the first predicted image. Here, the surgical parameter may include any operational parameters relating to a surgical technique and surgical instruments. The surgical parameter may be, for example, an amount of bone resection. The surgical parameter may be a parameter to be monitored or changed during a surgery, by a doctor or the like, or may be a parameter selected before a surgery.

If an operation instructing a change in the surgical parameter estimated by the estimator 27 is received, the generator 26 may generate a second predicted image obtained by performing processing corresponding to the change on the first predicted image. Accordingly, the information processing device 1a can facilitate trial and error by a doctor or the like in order to examine a surgical treatment method more suitable for the bones of the subject, and can support the application of the surgical treatment method more suitable for the bones of the subject.

Alternatively, the estimator 27 may output an estimation result regarding the strength of the affected bone of the subject from the first predicted image. The strength of the affected bone of the subject is important information for formulating a suitable surgical treatment method. The estimation result regarding the strength of the bone may be, for example, an estimation value of the bone density. By confirming such an estimation result, the doctor or the like can correctly determine whether the surgical treatment method suitable for the affected bone of the subject may be actually applied. As a result, the information processing device 1a can support the application of the surgical treatment method more suitable for the affected bones of the subject.

### Third Embodiment

### Configuration of Information Processing System 100b

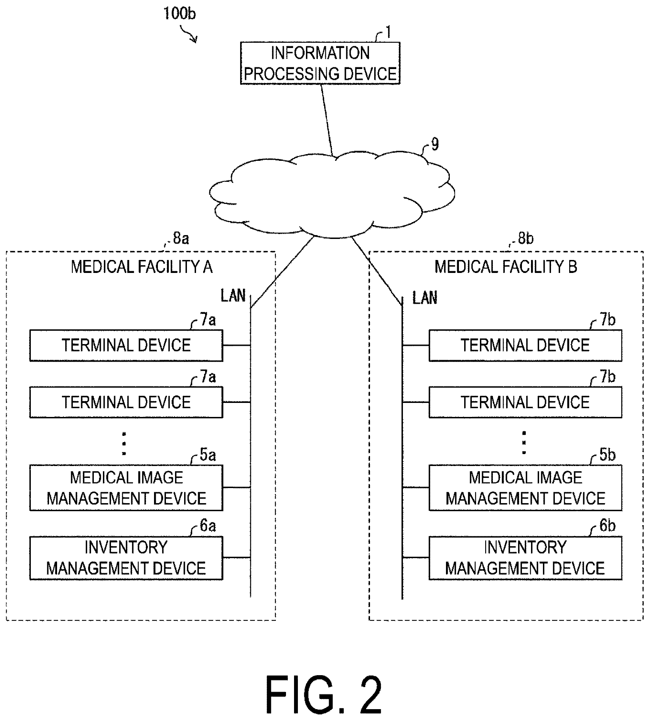

The information processing device 1 may be communicably connected to LANs each disposed in one of a plurality of the medical facilities 8 via a communication network 9, instead of a computer installed in the predetermined medical facility 8. FIG. 2 is a diagram illustrating a configuration example of the information processing system 100b according to another aspect of the present disclosure.

In addition to one or more terminal devices 7a, a medical image management device 5a and an inventory management device 6a may be communicably connected to the LAN in a medical facility 8a. To a terminal device 7b, a medical image management device 5b and an inventory management device 6b may be communicably connected to the LAN in a medical facility 8b. In the present disclosure, when no particular distinction is made between the medical facilities 8a and 8b, each of the medical facilities 8a and 8b is referred to as a "medical facility 8". When no particular distinction is made between the terminal devices 7a and 7b and between the medical image management devices 5a and 5b, each of the terminal devices 7a and 7b and each of the medical image management devices 5a and 5b are referred to as the "terminal device 7", the "medical image management device 5", and the "inventory management device 6".

FIG. 2 illustrates an example in which the LANs in the medical facility 8a and the medical facility 8b are connected to the communication network 9. The information processing device 1 is not limited to the configuration illustrated in FIG. 2, as long as the information processing device 1 is communicably connected to the medical image management device 5 and the inventory management device 6 in each medical facility via the communication network 9. For example, the information processing device 1 may be installed in the medical facility 8a or in the medical facility 8b.

In the information processing system 100b employing such a configuration, the information processing device 1 can acquire a medical image of a subject Pa examined in the medical facility 8a from the medical image management device 5a in the medical facility 8a. The information processing device 1 can acquire the holding status of implants, surgical instruments, and the like used in various types of medical procedures from the inventory management device 6a in the medical facility 8a. The information processing device 1 transmits at least any of the instrument information, the prediction information, the parameter information, and the implant information relating to the surgical treatment method suitable for the affected bones of the subject Pa, to the terminal device 7a installed in the medical facility 8a. The information processing device 1 transmits the instrument information, the prediction information, the parameter information, and the implant information relating to a surgical treatment method suitable for affected bone of the subject Pb, to the terminal device 7b.

When the medical facility 8a does not hold (or lacks) an implant usable in a surgical treatment method suitable for the affected bone of the subject Pa, the information processing device 1 may transmit at least any of the following information to the terminal device 7a.
- Information on an implant ordering procedure.
- Information on an expected delivery date when an implant is ordered.
A timing when the surgical treatment method is applied to the subject Pa may be influenced by the holding status of the implant in the medical facility 8a. If an implant to be used is out of inventory in the medical facility 8a, it is necessary to order the implant and wait until the implant is delivered. The information processing device 1 outputs the information as described above to support the planning of the surgical treatment suitable for the affected bones of the subject.

### Fourth Embodiment

An information processing device 1c and the information processing system 100c including the information processing device 1c according to a fourth embodiment will be described below. In the present embodiment, the information processing device 1c outputs estimated information of bone state information on a future or past bone state of a subject, and the estimated information is estimated using a learning model, based on input information including medical information of the subject. The medical information of the subject includes first medical information including a medical image showing a bone of the subject and second medical information including feature information of the subject at a time point different from a time point at which the medical image is captured. The learning model has been trained using training data including a plurality of pieces of information each including a medical image showing a bone of a predetermined person and feature information of the predetermined person at a time point different from a time point at which the medical image is captured.

### Configuration of Information Processing System 100c

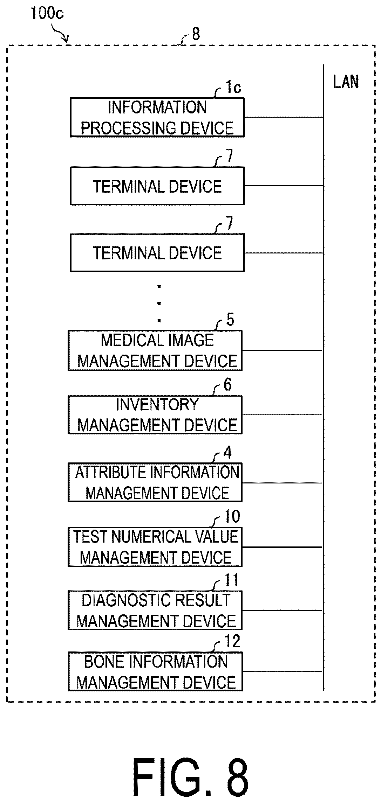

First, a configuration of the information processing system 100c according to the present embodiment will be described with reference to FIG. 8. FIG. 8 is a diagram illustrating a configuration example of the information processing system 100c in the medical facility 8 in which the information processing device 1c is introduced.

The information processing system 100c includes the information processing device 1c and the one or more terminal devices 7 communicably connected to the information processing device 1c. The information processing device 1c estimates bone state information on a future or past bone state of a subject, from medical information that includes first medical information including a medical image showing a bone of the subject and second medical information including feature information of the subject at a time point different from a time point when the medical image is captured. In the following description, an example in which bone state information on a future bone state of a subject is estimated (predicted) will be described, and the "bone state information on a future bone state" will also be simply referred to as "future bone state information". The information processing device 1c is a computer configured to transmit the estimated information of the future bone state information of the subject to the terminal device 7. The information processing device 1c may be installed on a cloud. In such a case, the terminal device 7 transmits the first medical information to the information processing device 1c on the cloud via the communication network, and receives the estimated information estimated by the information processing device 1c via the communication network.

In addition to the information processing device 1c and the terminal device 7, the medical image management device 5, an attribute information management device 4, the inventory management device 6, a test numerical value management device 10, a diagnostic result management device 11, and a bone information management device 12 may be communicably connected to the LANs in the medical facility 8.

The medical image management device 5 is a computer functioning as a server for managing medical images taken in the medical facility 8. The medical image management device 5 may be installed, for example, in the medical facility 8 or may be a cloud. When the medical image management device 5 is a cloud, the medical image can be acquired via a communication network. The medical image may be an image same as, and/or similar to the medical image used in the first to third embodiments. The medical image in the present embodiment may be any of an X-ray image, a CT image, an MRI image, an ultrasonic image captured by an ultrasonic diagnostic apparatus, a PET image, and a DXA image. The information processing device 1c may acquire a medical image showing a bone of the subject, from the medical image management device 5.

The attribute information management device 4 is a computer functioning as a server for managing attribute information of the subject. The attribute information includes at least one selected from the group consisting of an age, a sex, a height, a weight, a race, lifestyle habit information, medication information, occupation information, blood test information, urine test information, saliva test information, a medical history, a family medical history of the subject, genetic information, menopause information, items of FRAX, and menopause estimation estimated based on hormone information. The lifestyle habit may be, for example, a sleep time, a wake-up time, a sleep time, a daily exercise amount, a meal content, a meal time, a meal time period, and a blood sugar level. The meal content includes, for example, at least one selected from the group consisting of a meal name, an ingested food material, and an intake amount. The meal content may be an estimated intake including at least one selected from the group consisting of, for example, calcium, vitamin B, vitamin D, and vitamin K. For example, a designated value estimated from a parameter acquired by a wearable device may be employed for the blood glucose level.

The medication information may include information such as a name of a medication, a taken amount of the medication, and a period of taking the medication. The information on the medication being taken may include information on a steroid agent being used. The blood test information may be information on the result of at least any of, for example, a biochemical test, a glucose metabolism test, and an endocrine test.

The test numerical value management device 10 is a computer functioning as a server for managing a test numerical value obtained by a test taken in the medical facility 8. The test numerical value includes at least one selected from the group consisting of the bone density, Kellgren-Lawrence (KL) classification, a bone morphology angle, a muscle mass, Mini Mental State Examination (MMSE), a blood test numerical value, a hepatic function marker, a uric acid level, and a malignant tumor marker. The information processing device 1c may acquire the test numerical value of the subject from the test numerical value management device 10.

The diagnostic result management device 11 is a computer functioning as a server for managing diagnostic results from a diagnosis in the medical facility 8. The information processing device 1c may acquire the diagnostic result of the subject from the diagnostic result management device 11.

The bone information management device 12 is a computer functioning as a server for managing bone information acquired in the medical facility 8. The information processing device 1c may acquire the past bone information of the subject from the bone information management device 12.

### Configuration of Information Processing Device 1c

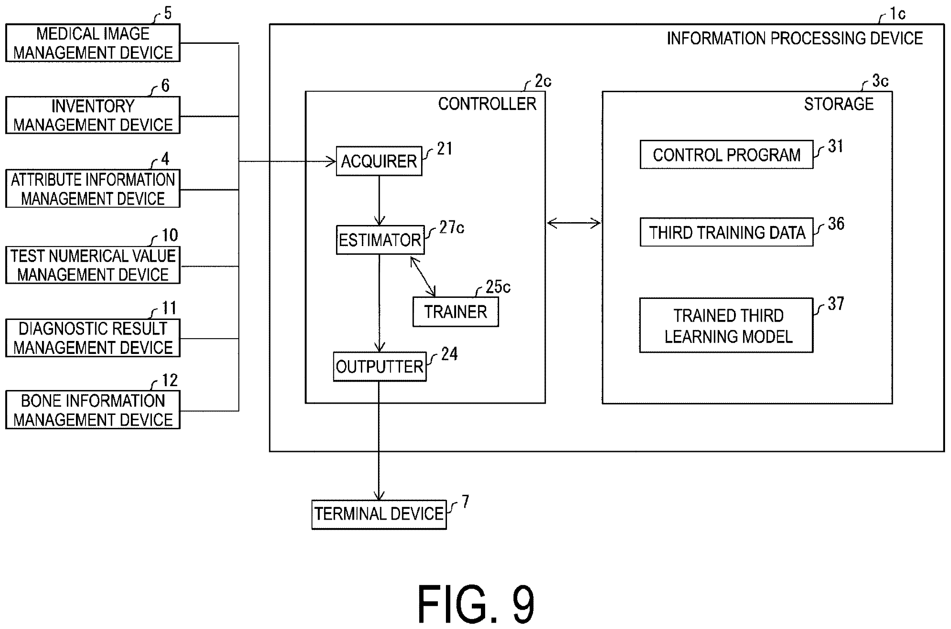

A configuration of the information processing device 1c applied to the information processing system 100c illustrated in FIG. 8 is described with reference to FIG. 9. FIG. 9 is a block diagram illustrating an example of a configuration example of the information processing device 1c.

The information processing device 1c includes a controller 2c that integrally controls each component of the information processing device 1c, and a storage 3c that stores various types of data to be used by the controller 2c. The controller 2c includes the acquirer 21, an estimator 27c, the outputter 24, and a trainer 25c. The estimator 27 described in the second embodiment may be the estimator 27c described in the present embodiment. The storage 3c stores third training data 36 and a trained third learning model 37 in addition to the control program 31, which is a program for executing various types of controls of the information processing device 1c.

### Acquirer 21

In the present embodiment, the acquirer 21 acquires input information including medical information of a subject. The input information is data input to the estimator 27c. In the present embodiment, the input information includes first medical information including a medical image showing a bone of the subject and second medical information including feature information of the subject at a time point different from a time point at which the medical image is captured. In the following description, a medical image showing a bone of the subject and being included in the first medical information may be referred to as a first medical image. The second medical information may be information at a time point when a period of, for example, 15 days, one month, three months, six months, one year, three years, or five years has elapsed from a time point when the medical image of the first medical information is captured. The second medical information may be information before or after the time point at which the medical image included in the first medical information is captured.

The feature information included in the second medical information includes at least one selected from the group consisting of a medical image of the subject, past bone information, attribute information, a test numerical value, and a diagnostic result. In the following description, a medical image showing a bone of the subject and being included in the second medical information may be referred to as a second medical image. The second medical information may be information at a plurality of time points or at one time point.

For example, the acquirer 21 may acquire a first medical image and/or a second medical image from the medical image management device 5, attribute information of the subject from the attribute information management device 4, a test numerical value of the subject from the test numerical value management device 10, a diagnostic result of the subject from the diagnostic result management device 11, or past bone information of the subject from the bone information management device 12. For example, the acquirer 21 acquires, as input information, an X-ray image captured at a first time point as first medical information and an X-ray image captured at a time point different from the first time point, for example, at a second time point before the first time point as second medical information. The acquirer 21 may further acquire, as the input information, an X-ray image captured at a third time point before the second time point as the second medical information.

The first medical information may include the same information element as the feature information included in the second medical information. In other words, the first medical information may include the same information as the feature information included in the second medical information, in addition to the first medical image. For example, when the second medical information includes the height information of the subject as the feature information, the first medical information may include, in addition to the first medical image, the height information of the subject at a time point when the first medical image is captured.

### Estimator 27c

The estimator 27c estimates future bone state information of the subject by inputting the input information acquired by the acquirer 21 to the third learning model 37. Here, the third learning model 37 is trained in advance using the third training data 36. The third training data 36 is data including medical information of a predetermined person. The predetermined person may be a patient suffering from a bone-related disease or a person not suffering from a disease. The medical information included in the third training data 36 may include a plurality of pieces of information including a medical image showing a bone of a predetermined person and feature information of the predetermined person at a time point different from a time point at which the medical image is captured.

The estimator 27c may estimate future or past bone state information of the subject by inputting, to the third learning model 37, as the input information, input information including first medical information including a medical image showing a bone of the subject and second medical information including feature information of the subject at a time point different from a time point at which the medical image is captured. By inputting the input information to the third learning model 37, the estimator 27c may estimate, as the bone state information, at least one selected from the group consisting of the probability of occurrence of a future or past fracture of the subject, a time point of occurrence of a future or past fracture in the subject (for example, four to five years later), a future or past bone density of the subject, and a future or past bone quality of the subject. More specifically, when the bone state information is the bone density, the estimator 27c can estimate how bone density was when the past first medical information was acquired, for example, even if the bone density was not measured in the past. The estimator 27c may estimate the bone density of the subject at a plurality of future and past time points from the first medical information. The estimator 27c may estimate the transition of the bone density of the subject from a certain time point in the past to a certain time point in the future. The information processing device 1c may graphically display the estimated transition on the display.

The estimator 27c may estimate, as the bone state information, what bone state information will be achieved or how bone state information will change, as a consequence of treatment for the subject. More specifically, when the bone state information is the future bone density of the subject, the estimator 27c may estimate, as the bone state information, the bone density or a change in the bone density that will be achieved as a consequence of treatment for the subject. The estimator 27c may estimate the content of the treatment in addition to the bone state information. The information processing device 1c may display the estimated treatment content on the display. The treatment can include, for example, taking medications, taking nutrients, or improving a lifestyle habit, for a predetermined period. The medication may include information on a medication having at least any of an effect on bone formation and an effect on bone resorption. Examples of the medication having an effect on bone formation may include, but are not limited to, an activated vitamin D3 preparation (for example, Calcitriol, Eldecalcitol, or Alfacalcidol), Teriparatide Acetate, Teriparatide (recombinant), and the like. Examples of the medication having an effect on bone resorption may include, but are not limited to, a calcitonin preparation, a bisphosphonate preparation, and an anti-RANKL monoclonal antibody.

The third learning model 37 is an operation model used by the estimator 27c in executing operations, based on the input data. The third learning model 37 is generated by the trainer 25c executing machine learning using the third training data 36, which will be described below, on an untrained neural network. Here, the third learning model 37 may also be applied to an animal other than a human. In such a case, the "predetermined person" in the third training data 36 may be a biological species of the same type as the "subject". That is, the information processing device 1c according to the present disclosure can also estimate the onset and/or progression of diseases of an animal other than a human. A specific example of the third training data 36, a configuration of the neural network, and the training processing will be described below.

### Outputter 24

The outputter 24 transmits information estimated by the estimator 27c to the terminal device 7. The information processing device 1c may include a display (not illustrated). In such a case, the outputter 24 causes the display to display the information estimated by the estimator 27c.

### Trainer 25c

The trainer 25c controls training processing for an untrained neural network. The trainer 25c creates a trained neural network functioning as the estimator 27c by executing the training processing on the untrained neural network. The third training data 36 (described below) is used for such a training. A specific example of training executed by the trainer 25c will be described below.

### Configuration of Estimator 27c

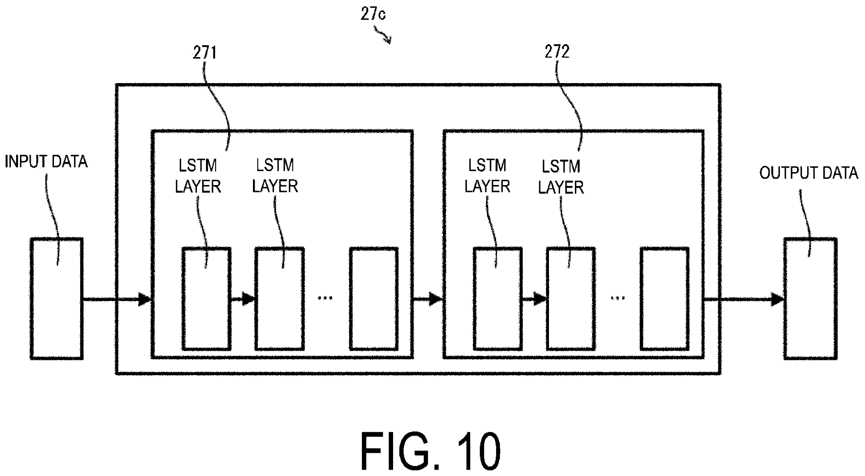

A configuration of the estimator 27c will be described below with reference to FIG. 10. The configuration illustrated in FIG. 10 is merely an example and the configuration of the estimator 27c is not limited thereto.

As illustrated in FIG. 10, the estimator 27c performs operations based on the third learning model 37 on the input data input to the input layer 271 to output output data from the output layer 272. In the present embodiment, the output data is future bone state information of the subject. The future bone state information may be, for example, a future probability of occurrence of a bone fracture of the subject, a future bone density of the subject, or a future bone quality of the subject.

The estimator 27c of FIG. 10 includes a neural network including the input layer 271 and the output layer 272. FIG. 10 illustrates a case where the neural network is an LSTM; however, the neural network is not limited thereto. For example, the neural network may be a ConvLSTM network in which CNN and LSTM are combined. The input layer 271 can extract a feature amount for a change in the input data. The output layer 272 can calculate a new feature amount, based on the feature amount extracted in the input layer 271 and the temporal change and the initial value of the input data. Each of the input layer 271 and the output layer 272 includes a plurality of LSTM layers. Each of the input layer 271 and the output layer 272 may include three or more LSTM layers.

### Training Processing by Trainer 25c

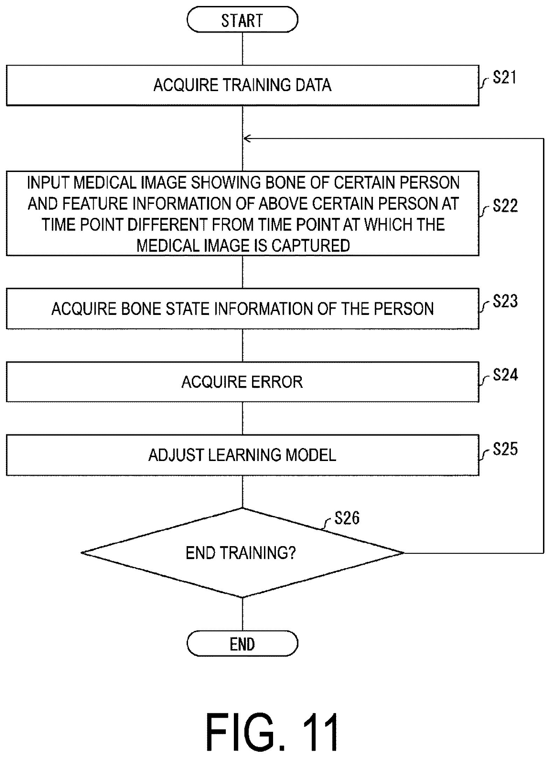

The training processing for generating the third learning model 37 will be described below with reference to FIG. 11. FIG. 11 is a flowchart illustrating an example of a flow of training processing by the trainer 25c.

The trainer 25c acquires the third training data 36 from the storage 3c (step S21). The third training data 36 includes a plurality of pieces of information including bone state information on a future bone state of a predetermined person, and includes an explanatory variable and an objective variable to be input to the input layer 271. Here, in the present embodiment, the explanatory variable is a medical image showing a bone of a predetermined person and feature information of the predetermined person at a time point different from a time point at which the medical image is captured, and the objective variable is bone state information of each person. The third training data 36 may include content of treatment of a predetermined person. The treatment can include, for example, taking medications, taking nutrients, or improving a lifestyle habit, for a predetermined period. The medication may include information on a medication having at least any of an effect on bone formation and an effect on bone resorption. Examples of the medication having an effect on bone formation may include, but are not limited to, an activated vitamin D3 preparation (for example, Calcitriol, Eldecalcitol, or Alfacalcidol), Teriparatide Acetate, Teriparatide (recombinant), and the like. Examples of the medication having an effect on bone resorption may include, but are not limited to, a calcitonin preparation, a bisphosphonate preparation, and an anti-RANKL monoclonal antibody.

The trainer 25c inputs a medical image showing a bone of a certain person (referred to as a person A) and feature information of the person A at a time point different from a time point at which the medical image is captured, to the input layer 271 as information including future bone state information (step S22).

The trainer 25c acquires output data regarding the bone state information of the person A from the output layer 272 (step S23). The output data contains the same contents as the objective variable of the third training data 36. In FIG. 11, the order of step S22 and step S23 may be reversed. Alternatively, in FIG. 11, step S22 and step S23 may be executed simultaneously.

The trainer 25c acquires an objective variable for the person A included in the third training data 36. The trainer 25c compares the output data acquired in step S23 with the objective variable for the person A, calculates an error (step S24), and adjusts the learning model being trained so as to reduce the error (step S25).

Any known method is applicable to the adjustment of the learning model being trained. For example, an error backpropagation method may be employed as a method for adjusting the learning model. The adjusted learning model becomes a new learning model, and in subsequent operations, the estimator 27c uses the new learning model. In the adjustment stage of the learning model, the parameters (for example, a filter coefficient and a weighting factor) used in the estimator 27c may be adjusted.

If the error is not within the predetermined range, and if the explanatory variables for all the persons contained in the third training data 36 are not input (NO in step S26), the trainer 25c returns to step S22 and repeats the training processing. If the error is within the predetermined range, and if the explanatory variables for all the patients contained in the third training data 36 are input (YES in step S26), the trainer 25c ends the training processing.

When the training processing as described above is adopted, the estimator 27c can estimate the future bone state information of the subject from medical information that includes first medical information including a medical image showing a bone of the subject and second medical information including feature information of the subject at a time point different from a time point at which the medical image is captured.

In one aspect of the present disclosure, the third learning model 37 may be a learning model trained using training data including medical information of a predetermined person acquired at a plurality of different time points. For example, the third learning model 37 may be a learning model trained using training data including medical images of a predetermined person acquired at a plurality of different time points as an explanatory variable and including the bone density of the predetermined person acquired at the plurality of different time points as an objective variable. Accordingly, the estimation accuracy may be improved by inputting the medical information of the subject acquired at at least two time points different from each other to the third learning model 37.

### Processing Executed by Information Processing Device 1c

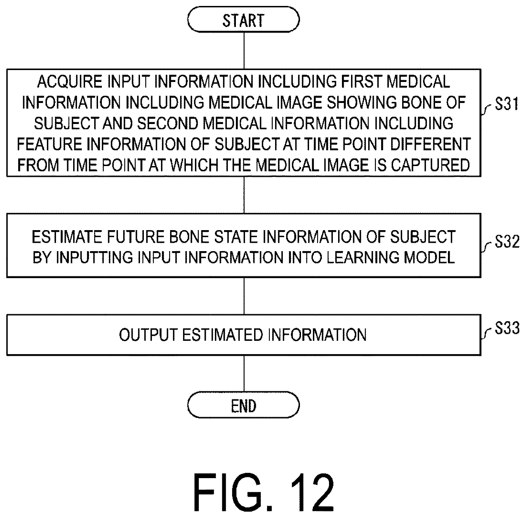

A flow of processing executed by the information processing device 1c will be described below with reference to FIG. 12. FIG. 12 is a flowchart illustrating an example of a flow of processing executed by the information processing device 1c.

First, the acquirer 21 acquires medical information that includes first medical information including a medical image showing a bone of a subject and second medical information including feature information of the subject at a time point different from a time point at which the medical image is captured (step S31: acquiring step).

The estimator 27c inputs the acquired medical information to the third learning model 37 to estimate future bone state information of the subject (step S32: estimating step).

The outputter 24 outputs each piece of information including the bone state information estimated in step S32 (step S33: outputting step).

According to such a configuration, the information processing device 1c and the information processing system 100c estimate the future bone state information of the subject from the medical information that includes the first medical information including the medical image showing a bone of the subject and the second medical information including the feature information of the subject at a time point different from a time point at which the medical image is captured. Accordingly, the estimation accuracy may be improved as compared with a case where the bone state information is estimated using the input information including the medical information acquired at one time point.

The estimator 27c may estimate the bone state information of the subject after a time shorter than a time interval between any two time points of the time point at which the first medical information is acquired and the at least one time point at which the second medical information is acquired. Accordingly, the bone state information may be more accurately estimated based on the change information of the state of the subject in a time interval longer than the time interval from the current time point to the estimation time point.

The estimator 27c may estimate the future bone state information of the subject by inputting, to the third learning model 37, input information that includes medical information at three or more time points, that is, first medical information including a medical image showing a bone of the subject and second medical information including feature information of the subject at two or more time points different from the time point at which the medical image is captured. Accordingly, the acceleration degree of the temporal change of the bone state up to the current time point can be known, and the bone state information at the estimation time point can be estimated with higher accuracy.

The estimator 27c may estimate whether the subject is menopausal from the attribute information of the subject and the degree of acceleration of the temporal change in the state of bones at three or more time points. In such a case, the estimator 27c may use the estimated menopause information as the menopause information of the attribute information of the subject.

If the second medical information is a medical image, a site included in the medical image of the second medical information may be different from a site included in the medical image of the first medical information. For example, the site included in the image of the first medical information may be the chest, and the medical image of the second medical information may be a dental medical image. As a result, images captured in a medical examination or a dental treatment can be flexibly used, and versatility is improved.

The estimator 27c may estimate the bone state information of the subject at a time (the time point is referred to as an estimation time point) after a time longer than a time interval (hereinafter referred to as a first time interval) between the earliest time point and the latest time point among the time point at which the first medical information is acquired and the at least one time point at which the second medical information is acquired. As described above, the information processing system 100c according to the present disclosure estimates the future bone state information of the subject using the first medical information and the second medical information acquired at different time points, and thus, the future bone state information of the subject within the first time interval from the current time point may be estimated with high accuracy. Therefore, in estimating the bone state information of the subject after the first time interval from the current time point, the bone state information can be estimated using the future bone state information of the subject by the first time interval from the current time point estimated with high accuracy. As a result, the bone state information of the subject may be more accurately estimated compared to a case where the bone state information is estimated using medical information at one time point.

When the second medical information acquired by the acquirer 21 includes medical information at two or more time points and data of any of the second medical information at the two or more time points and the first medical information is anomalous data, the estimator 27c may input data excluding the anomalous data to the third learning model 37. The anomalous data is, for example, a test numerical value being caused by a test error or the like and being medically impossible. According to such a configuration, the possibility that erroneous information is input to the third learning model 37 may be reduced to improve the estimation accuracy. In such a case, for example, the estimator 27c may identify, as thresholds, a lower limit value and an upper limit value of values that are medically possible as test numerical values, and may input, to the third learning model 37, data excluding data outside the range of the thresholds.

When the second medical information acquired by the acquirer 21 includes medical information at two or more time points and data of any of the second medical information at the two or more time points and the first medical information is anomalous data, the estimator 27c may replace the anomalous data with data obtained by approximating the anomalous data to other data to be input into the third learning model 37. According to such a configuration, the possibility that erroneous information is input to the third learning model 37 may be reduced to improve the estimation accuracy.

Menopause is known to result in significant reduction of bone density. Therefore, when the subject is a woman who is not menopausal at the current time point, the estimator 27c may output the first estimation result on the assumption that the subject is menopausal and/or the second estimation result on the assumption that the subject is not menopausal. Accordingly, by taking into account both cases before and after menopause, an estimation result with higher accuracy may be output. When the subject is a woman who is not menopausal at the current time point, the estimator 27c may simultaneously output the first estimation result on the assumption that the subject is menopausal and the second estimation result on the assumption that the subject is not menopausal.

### Fifth Embodiment

### Configuration of Information Processing System 100d

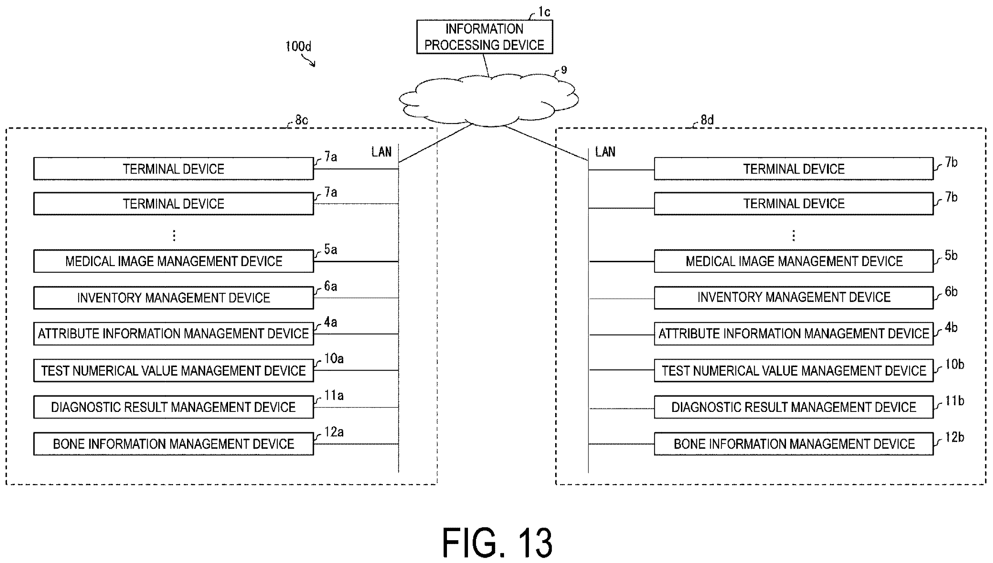

The information processing device 1c may be communicably connected to LANs each disposed in one of the plurality of medical facilities 8 via the communication network 9, instead of a computer installed in the predetermined medical facility 8. FIG. 13 is a diagram illustrating a configuration example of the information processing system 100d according to yet another aspect of the fourth embodiment.

In addition to the one or more terminal device 7a, the medical image management device 5a, the inventory management device 6a, an attribute information management device 4a, a test numerical value management device 10a, a diagnostic result management device 11a, and a bone information management device 12a may be communicably connected to the LAN in a medical facility 8c. In addition to the terminal device 7b, the medical image management device 5b, the inventory management device 6b, an attribute information management device 4b, a test numerical value management device 10b, a diagnostic result management device 11b, and a bone information management device 12b may be communicably connected to the LAN in a medical facility 8d. In the present disclosure, when no particular distinction is made between the medical facilities 8c and 8d, each of the medical facilities 8c and 8d is referred to as the "medical facility 8". When no particular distinction is made between the terminal devices 7a and 7b, between the medical image management devices 5a and 5b, between the attribute information management devices 4a and 4b, between the test numerical value management devices 10a and 10b, between the diagnostic result management devices 11a and 11b, and between the bone information management devices 12a and 12b, each of them is also referred to as the "terminal device 7", the "medical image management device 5", the "attribute information management device 4", the "test numerical value management device 10", the "diagnostic result management device 11", and the "bone information management device 12", respectively.

FIG. 13 illustrates an example in which the LANs in the medical facility 8c and the medical facility 8d are connected to the communication network 9. The information processing device 1c is not limited to the configuration illustrated in FIG. 13 as long as the information processing device 1c is communicably connected to the medical image management device 5, the inventory management device 6, the attribute information management device 4, the test numerical value management device 10, the diagnostic result management device 11, and the bone information management device 12 in each medical facility via the communication network 9. For example, the information processing device 1c may be installed in the medical facility 8c or in the medical facility 8d.

In the information processing system 100d employing such a configuration, the information processing device 1c can acquire a medical image of a subject Pa examined in the medical facility 8c, the attribute information, the test numerical value, and the diagnostic result, from the medical image management device 5a, the attribute information management device 4a, the test numerical value management device 10a, and the diagnostic result management device 11a in the medical facility 8c, respectively. The information processing device 1c transmits the estimation result of the future bone state information of the subject Pa to the terminal device 7a installed in the medical facility 8c. The information processing device 1c transmits the future bone state information of a subject Pb to the terminal device 7b.

### Supplementary Note

The information processing device 1 may not include all the functional blocks included in the controller 2 of the information processing device 1. For example, in the information processing system 100a, the terminal device 7 may have the function of the acquirer 21, and the information processing device 1 may receive the input information from the terminal device 7. Similarly, in each of the information processing systems 100b, 100c, and 100d, the information processing device 1 may receive input information from the terminal device 7.

For example, the information processing systems 100a, 100b, 100c, and 100d may include the information processing device 1 in which the trained first learning model 34, the second learning model 35, and/or the third learning model 37 trained by another computer that is different from the information processing device 1 and has the function of the trainer 25 are/is installed.

For example, in the information processing systems 100a, 100b, 100c, and 100d, another computer other than the information processing device 1 or the terminal device 7 may have the function of the generator 26. In the information processing systems 100a, 100b, 100c, and 100d, another computer other than the information processing device 1 or the terminal device 7 may have the function of the estimators 27, 27c.

### Example of Implementation using Software

The functions of the information processing devices 1, 1a, and 1c (hereinafter, referred to as the "device") can be implemented by a program for causing a computer to function as the device and for causing the computer to function as each control block (particularly, each component included in the controllers 2, 2a, and 2c) of the device.

In such a case, the above device includes a computer including at least one control device (for example, a processor) and at least one storage device (for example, a memory) as hardware for executing the above program. By executing the program by the control device and the storage device, the functions described in the embodiments are implemented.

The program may be recorded on one or more computer-readable non-transitory recording media. The recording media may be or need not be included in the device. In the latter case, the program may be supplied to the device via any wired or wireless transmission medium.

Some or all of the functions of the control blocks can be implemented by logic circuits. For example, an integrated circuit in which logic circuits functioning as the control blocks are formed is also included in the scope of the present disclosure. In addition to this, for example, a quantum computer can implement the functions of the control blocks.

The processing described in the embodiments may be executed by artificial intelligence (AI). In such a case, the AI may operate on the control device or may operate on another device (such as an edge computer or a cloud server).

The invention according to the present disclosure has been described above based on various drawings and example. However, the invention according to the present disclosure is not limited to each embodiment described above. That is, the invention according to the present disclosure can be modified in various ways within the scope described in the present disclosure, and embodiments obtained by combining technical means disclosed in the different embodiments as appropriate are also included in the technical scope of the invention according to the present disclosure. In other words, it should be noted that a person skilled in the art can easily make various variations or modifications based on the present disclosure. It should also be noted that these variations or modifications are included in the scope of the present disclosure.

### Conclusion

An information processing system according to a first aspect of the present disclosure includes an acquirer, an identifier, and an outputter. The acquirer is configured to acquire input information including a medical image showing an affected bone of a subject. The identifier includes a first learning model trained using first training data. The first training data includes bone information on at least any of a bone density and a bone quality of a bone before application of a surgical treatment method including an implant embedding procedure, a shape feature, and treatment information on a surgical treatment method applied to the bone, of each of a plurality of patients. The outputter is configured to output at least any of instrument information, prediction information, parameter information, and implant information each identified by the identifier based on the input information. The instrument information indicates a surgical instrument to be used in a surgical treatment method suitable for the affected bone of the subject, the prediction information indicates a prediction of a state of the affected bone of the subject after application of the surgical treatment method, the parameter information indicates a parameter selectable when the surgical treatment method is applied to the affected bone of the subject, and the implant information is information on an implant embeddable in the affected bone of the subject.

An information processing system according to a second aspect of the present disclosure is the information processing system of the first aspect in which the implant information may include inventory information of the implant embeddable in the affected bone of the subject.

An information processing system according to a third aspect of the present disclosure is the information processing system of the first or second aspect in which the first training data may include, as the treatment information, a content and a result of a surgical treatment method applied to an affected bone of each of the plurality of patients.

An information processing system according to a fourth aspect of the present disclosure is the information processing system of any one of the first to third aspects in which the first training data may include information indicating a surgical treatment method applied to an affected bone of each of the plurality of patients, a medical image showing the affected bone of each of the plurality of patients before application of the surgical treatment method, and information on an implant embedded in the affected bone of each of the plurality of patients.

An information processing system according to a fifth aspect of the present disclosure is the information processing system of any one of the first to fourth aspects in which the first training data may include information on a surgical instrument used in a surgical treatment method applied to an affected bone of each of the plurality of patients.

An information processing system according to a sixth aspect of the present disclosure is the information processing system of any one of the first to fifth aspects in which the outputter may further output inventory information of the surgical instrument to be used in the surgical treatment method suitable for the affected bone of the subject, the surgical instrument having been identified by the identifier based on the input information.

An information processing system according to a seventh aspect of the present disclosure is the information processing system of any one of the first to sixth aspects in which the first training data may further include at least any of event information on an event that occurred during an implant embedding procedure for a bone of each of the plurality of patients and procedure information on a procedure performed in the implant embedding procedure.

An information processing system according to an eighth aspect of the present disclosure is the information processing system of any one of the first to seventh aspects in which the outputter may output the prediction information including a first predicted image generated from the medical image included in the input information, and the first predicted image may show an expected state of the affected bone of the subject after application of the surgical treatment method to the affected bone.

An information processing system according to a ninth aspect of the present disclosure is the information processing system of the eighth aspect in which the first predicted image may be an image generated from the medical image included in the input information by using a second learning model trained by using second training data including a medical image obtained by imaging an affected bone of a patient, to which the surgical treatment method suitable for the affected bone of the subject is applied, before and after the application of the surgical treatment method.

An information processing system according to a tenth aspect of the present disclosure is the information processing system of the eighth or ninth aspect which may output the parameter information including a surgical parameter estimated from a result of image analysis of the first predicted image, and the surgical parameter may be selectable by a medical personnel applying the surgical treatment method suitable for the affected bone of the subject to the subject.

An information processing system according to an eleventh aspect of the present disclosure is the information processing system of the tenth aspect which may further include a generator configured to generate, in response to an operation instructing a change of the estimated surgical parameter, a second predicted image obtained by performing processing corresponding to the change on the first predicted image.

An information processing system according to a twelfth aspect of the present disclosure is the information processing system of any one of the eighth to eleventh aspects which may further include an estimator configured to output an estimation result regarding strength of the affected bone of the subject from the first predicted image.

An information processing system according to a thirteenth aspect of the present disclosure is the information processing system of any one of the eighth to twelfth aspects in which the first predicted image may be a three-dimensional image obtained by conversion from a two-dimensional image.

An information processing system according to a fourteenth aspect of the present disclosure is the information processing system of any one of the first to thirteenth aspects in which the surgical treatment method may be at least any of an artificial joint replacement procedure and a dental implant surgery.

An information processing system according to a fifteenth aspect of the present disclosure is the information processing system of any one of the first to fourteenth aspects in which the surgical treatment method may be implementable by a surgical robot.

A method for controlling an information processing system according to a sixteenth aspect of the present disclosure includes acquiring input information including a medical image showing an affected bone of a subject, and outputting at least any of instrument information, prediction information, parameter information, and implant information each identified based on the input information and by using a first learning model trained using first training data. The instrument information indicates a surgical instrument to be used in a surgical treatment method suitable for the affected bone of the subject, the prediction information indicates a prediction of a state of the affected bone of the subject after application of the surgical treatment method, the parameter information indicates a parameter selectable when the surgical treatment method is applied to the affected bone of the subject, and the implant information is information on an implant embeddable in the affected bone of the subject. The first training data includes bone information on at least any of a bone density and a bone quality of a bone before application of a surgical treatment method including an implant embedding procedure, a shape feature, and treatment information on the surgical treatment method applied to the bone, of each of a plurality of patients.

A control program of the information processing device according to a seventeenth aspect of the present disclosure is a control program for causing a computer to function as the information processing system according to any of the first to fifteen aspects, and the control program causes the computer to function as the acquirer, the identifier, and the outputter.

A recording medium according to an eighteenth aspect of the present disclosure is a computer-readable recording medium on which the control program according to the seventeenth aspect is recorded.

### REFERENCE SIGNS

1, 1a Information processing device
21 Acquirer
23 Identifier
24 Outputter
26 Generator
27 Estimator
32 First training data
34 Trained first learning model (first learning model)
35 Trained second learning model (second learning model)
100a, 100b, 100c, and 100d Information processing system
S11 Acquiring step
S12 Identifying step
S13 Outputting step

## Claims

1. An information processing system comprising:
an acquirer configured to acquire input information comprising a medical image showing an affected bone of a subject;
an identifier comprising a first learning model trained using first training data, the first training data comprising bone information on at least any of a bone density and a bone quality of a bone before application of a surgical treatment method comprising an implant embedding procedure, a shape feature, and treatment information on a surgical treatment method applied to the bone, of each of a plurality of training subjects; and
an outputter configured to output at least any of instrument information, prediction information, parameter information, and implant information each identified by the identifier based on the input information, the instrument information indicating a surgical instrument to be used in a surgical treatment method suitable for the affected bone of the subject, the prediction information indicating a prediction of a state of the affected bone of the subject after application of the surgical treatment method, the parameter information indicating a parameter selectable when the surgical treatment method is applied to the affected bone of the subject, and the implant information on an implant embeddable in the affected bone of the subject.

2. The information processing system according to claim 1, wherein the implant information comprises inventory information of the implant embeddable in the affected bone of the subject.

3. The information processing system according to claim 1 or 2, wherein the first training data comprises, as the treatment information, a content and a result of a surgical treatment method applied to an affected bone of each of the plurality of training subjects.

4. The information processing system according to any one of claims 1 to 3, wherein the first training data comprises information indicating a surgical treatment method applied to an affected bone of each of the plurality of training subjects, a medical image showing the affected bone of each of the plurality of training subjects before application of the surgical treatment method, and information on an implant embedded in the affected bone of each of the plurality of training subjects.

5. The information processing system according to any one of claims 1 to 4, wherein the first training data comprises information on a surgical instrument used in a surgical treatment method applied to an affected bone of each of the plurality of training subjects.

6. The information processing system according to any one of claims 1 to 5, wherein the outputter further outputs inventory information of the surgical instrument to be used in the surgical treatment method suitable for the affected bone of the subject, the surgical instrument having been identified by the identifier based on the input information.

7. The information processing system according to any one of claims 1 to 6, wherein the first training data further comprises at least any of event information on an event during an implant embedding procedure for a bone of each of the plurality of training subjects and procedure information on a procedure performed in the implant embedding procedure.

8. The information processing system according to any one of claims 1 to 7, wherein the outputter outputs the prediction information comprising a first predicted image generated from the medical image comprised in the input information, the first predicted image showing an expected state of the affected bone of the subject after application of the surgical treatment method to the affected bone.

9. The information processing system according to claim 8, wherein the first predicted image is an image generated from the medical image comprised in the input information by using a second learning model trained by using second training data comprising a medical image obtained by imaging an affected bone of a training subject, of the plurality of training subjects, to which the surgical treatment method suitable for the affected bone of the subject is applied, before and after the application of the surgical treatment method.

10. The information processing system according to claim 8 or 9, wherein the outputter outputs the parameter information comprising a surgical parameter estimated from a result of image analysis of the first predicted image, the surgical parameter being selectable by a medical personnel applying the surgical treatment method suitable for the affected bone of the subject to the subject.

11. The information processing system according to claim 10, further comprising a generator configured to generate, in response to an operation instructing a change of the estimated surgical parameter, a second predicted image obtained by performing processing corresponding to the change on the first predicted image.

12. The information processing system according to any one of claims 8 to 11, further comprising an estimator configured to output an estimation result regarding strength of the affected bone of the subject, from the first predicted image.

13. The information processing system according to any one of claims 8 to 12, wherein the first predicted image is a three-dimensional image obtained by conversion from a two-dimensional image.

14. The information processing system according to any one of claims 1 to 13, wherein the surgical treatment method is at least any of an artificial joint replacement procedure and a dental implant surgery.

15. The information processing system according to any one of claims 1 to 14, wherein the surgical treatment method is implementable by a surgical robot.

16. A method for controlling an information processing system, comprising:
acquiring input information comprising a medical image showing an affected bone of a subject; and
outputting at least any of instrument information, prediction information, parameter information, and implant information each identified based on the input information and by using a first learning model trained using first training data, the instrument information indicating a surgical instrument to be used in a surgical treatment method suitable for the affected bone of the subject, the prediction information indicating a prediction of a state of the affected bone of the subject after application of the surgical treatment method, the parameter information indicating a parameter selectable when the surgical treatment method is applied to the affected bone of the subject, the implant information on an implant embeddable in the affected bone of the subject, and the first training data comprising bone information on at least any of a bone density and a bone quality of a bone before application of a surgical treatment method comprising an implant embedding procedure, a shape feature, and treatment information on the surgical treatment method applied to the bone, of each of a plurality of training subjects.

17. A control program for causing a computer to function as the information processing system according to any one of claims 1 to 15, wherein the control program causes the computer to function as the acquirer, the identifier, and the outputter.

18. A computer-readable recording medium on which the control program according to claim 17 is recorded.
